# EUROPEAN PATENT APPLICATION

(11) **EP 1 092 768 A1**
(43) Date of publication of application: **18.04.2001**
(21) Application number: 99120592.3
(22) Date of filing: 16.10.1999
(51) Int. Cl.: C12N 15/10, A01K 67/027, C12N 15/90

(54) **Conditional gene trapping construct for the disruption of genes**

(71) Applicant: ARTEMIS Pharmaceuticals GmbH, 51063 Köln (DE); FrankGen Biotechnologie AG, 40470 Düsseldorf (DE)
(72) Inventor: Kühn, Ralf, Dr., 50937 Köln (DE); von Melchner, Harald, Prof. Dr., 64380 Rossdorf (DE)
(74) Representative: Helbing, Jörg, Dr. Dipl.-Chem.

(57) **Abstract**

The present invention relates to a gene trapping construct which causes conditional mutations in genes, and the use of this gene trapping construct to mutationally inactivate all cellular genes. In addition the invention relates to a cell, preferably a mammalian cell which contains the above mentioned construct. Moreover, the invention relates to the use of said cell for identification and/or isolation of genes and for the creation of transgenic organisms to study gene function at various developmental stages, including the adult. In conclusion, the present invention provides a process which enables a temporally and/or spatially restricted inactivation of all genes that constitute a living organism.

SEQUENCE LISTING

<110> Artemis Pharmaceuticals GmbH
      FrankGen Biotechnologie AG

<120> Conditional gene trapping construct for the disruption
      of genes

<130> 992228ep/JH/ml

<140>
<141>

<160> 10

<170> PatentIn Ver. 2.1

<210> 1
<211> 120
<212> DNA
<213> Adenovirus

<400> 1
gtgacctgca cgtctagggc gcagtagtcc agggtttcct tgatgatgtc atacttatcc 60
tgtccctttt ttttccacag ctcgcggttg aggacaaact cttcgcggtc tttccagtac 120

<210> 2
<211> 34
<212> DNA
<213> Artificial Sequence

<220>
<223> Description of Artificial Sequence: mutant loxP
      site - lox66

<400> 2
ataacttcgt atagcataca ttatacgaac ggta                                34

<210> 3
<211> 34
<212> DNA
<213> Artificial Sequence

<220>
<223> Description of Artificial Sequence:mutant loxP
      site - lox71

<400> 3
taccgttcgt atagcataca ttatacgaag ttat                                34

<210> 4
<211> 34
<212> DNA
<213> Artificial Sequence

<220>
<223> Description of Artificial Sequence:double mutant
      loxP site - lox66/71

<400> 4
taccgttcgt atagcataca ttatacgaac ggta                                34

<210> 5
<211> 34
<212> DNA
<213> Bacteriophage P1

<400> 5

ataacttcgt atagcataca ttatacgaag ttat 34

<210> 6
<211> 277
<212> DNA
<213> Artificial Sequence

<220>
<223> Description of Artificial Sequence:poly A signal
sequence

<400> 6
tctagagctc gctgatcagc ctcgactgtg ccttctagtt gccagccatc tgttgtttgc 60
ccctcccccg tgccttcctt gaccctggaa ggtgccactc ccactgtcct ttcctaataa 120
aatgaggaaa ttgcatcgca ttgtctgagt aggtgtcatt ctattctggg gggtggggtg 180
gggcaggaca gcaaggggga ggattgggaa gacaatagca ggcatgctgg ggatgcggtg 240
ggctctatgg cttctgaggc ggaaagaacc agctggg 277

<210> 7
<211> 48
<212> DNA
<213> Artificial Sequence

<220>
<223> Description of Artificial Sequence:FRT site

<400> 7
gaagttccta tacttctaga agaataggaa cttccgaata ggaacttc 48

<210> 8
<211> 585
<212> DNA
<213> ECM virus

<400> 8

ctctccctcc ccccccccta acgttactgg ccgaagccgc ttggaataag gccggtgtgc 60

gtttgtctat atgtgatttt ccaccatatt gccgtctttt ggcaatgtga gggcccggaa 120

acctggccct gtcttcttga cgagcattcc taggggtctt tcccctctcg ccaaaggaat 180

gcaaggtctg ttgaatgtcg tgaaggaagc agttcctctg gaagcttctt gaagacaaac 240

aacgtctgta gcgacccttt gcaggcagcg gaacccccca cctggcgaca ggtgcctctg 300

cggccaaaag ccacgtgtat aagatacacc tgcaaaggcg gcacaacccc agtgccacgt 360

tgtgagttgg atagttgtgg aaagagtcaa atggctctcc tcaagcgtat tcaacaaggg 420

gctgaaggat gcccagaagg taccccattg tatgggatct gatctggggc ctcggtgcac 480

atgctttaca tgtgtttagt cgaggttaaa aaaacgtcta ggccccccga accacgggga 540

cgtggttttc ctttgaaaaa cacgatgata agcttgccac aaccc 585

<210> 9

<211> 600

<212> DNA

<213> Artificial Sequence

<220>

<223> Description of Artificial Sequence:Puromycin
      resistance gene

<400> 9

atgaccgagt acaagcccac ggtgcgcctc gccacccgcg acgacgtccc ccgggccgta 60

cgcaccctcg ccgccgcgtt cgccgactac cccgccacgc gccacaccgt cgacccggac 120

cgccacatcg agcgggtcac cgagctgcaa gaactcttcc tcacgcgcgt cgggctcgac 180

atcggcaagg tgtgggtcgc ggacgacggc gccgcggtgg cggtctggac cacgccggag 240

agcgtcgaag cgggggcggt gttcgccgag atcggcccgc gcatggccga gttgagcggt 300

tcccggctgg ccgcgcagca acagatggaa ggcctcctgg cgccgcaccg gcccaaggag 360

cccgcgtggt tcctggccac cgtcggcgtc tcgcccgacc accagggcaa gggtctgggc 420

agcgccgtcg tgctccccgg agtggaggcg gccgagcgcg ccggggtgcc cgccttcctg 480

gagacctccg cgccccgcaa cctccccttc tacgagcggc tcggcttcac cgtcaccgcc 540

gacgtcgagt gcccgaagga ccgcgcgacc tggtgcatga cccgcaagcc cggtgcctga 600

<210> 10

<211> 3652

<212> DNA

<213> Escherichia coli

<400> 10

atgagcgaaa aatacatcgt cacctgggac atgttgcaga tccatgcacg taaactcgca 60

agccgactga tgccttctga acaatggaaa ggcattattg ccgtaagccg tggcggtctg 120

gtaccggtgg gtgaagacca gaaacagcac ctcgaactga gccgcgatat tgcccagcgt 180

ttcaacgcgc tgtatggcga gatcgatccc gtcgttttac aacgtcgtga ctgggaaaac 240

cctggcgtta cccaacttaa tcgccttgca gcacatcccc ctttcgccag ctggcgtaat 300

agcgaagagg cccgcaccga tcgcccttcc caacagttgc gcagcctgaa tggcgaatgg 360

cgctttgcct ggtttccggc accagaagcg gtgccggaaa gctggctgga gtgcgatctt 420

cctgaggccg atactgtcgt cgtcccctca aactggcaga tgcacggtta cgatgcgccc 480

atctacacca acgtaaccta tcccattacg gtcaatccgc cgtttgttcc cacggagaat 540

ccgacgggtt gttactcgct cacatttaat gttgatgaaa gctggctaca ggaaggccag 600

acgcgaatta tttttgatgg cgttaactcg gcgtttcatc tgtggtgcaa cgggcgctgg 660

gtcggttacg gccaggacag tcgtttgccg tctgaatttg acctgagcgc atttttacgc 720

gccggagaaa accgcctcgc ggtgatggtg ctgcgttgga gtgacggcag ttatctggaa 780

gatcaggata tgtggcggat gagcggcatt ttccgtgacg tctcgttgct gcataaaccg 840

actacacaaa tcagcgattt ccatgttgcc actcgcttta atgatgattt cagccgcgct 900

gtactggagg ctgaagttca gatgtgcggc gagttgcgtg actacctacg ggtaacagtt 960

tctttatggc agggtgaaac gcaggtcgcc agcggcaccg cgcctttcgg cggtgaaatt 1020

atcgatgagc gtggtggtta tgccgatcgc gtcacactac gtctgaacgt cgaaaacccg 1080

aaactgtgga gcgccgaaat cccgaatctc tatcgtgcgg tggttgaact gcacaccgcc 1140

gacggcacgc tgattgaagc agaagcctgc gatgtcggtt tccgcgaggt gcggattgaa 1200

aatggtctgc tgctgctgaa cggcaagccg ttgctgattc gaggcgttaa ccgtcacgag 1260

catcatcctc tgcatggtca ggtcatggat gagcagacga tggtgcagga tatcctgctg 1320

atgaagcaga acaactttaa cgccgtgcgc tgttcgcatt atccgaacca tccgctgtgg 1380

tacacgctgt gcgaccgcta cggcctgtat gtggtggatg aagccaatat tgaaacccac 1440

ggcatggtgc caatgaatcg tctgaccgat gatccgcgct ggctaccggc gatgagcgaa 1500

cgcgtaacgc gaatggtgca gcgcgatcgt aatcacccga gtgtgatcat ctggtcgctg 1560

gggaatgaat caggccacgg cgctaatcac gacgcgctgt atcgctggat caaatctgtc 1620

gatccttccc gcccggtgca gtatgaaggc ggcggagccg acaccacggc caccgatatt 1680

atttgcccga tgtacgcgcg cgtggatgaa gaccagccct tcccggctgt gccgaaatgg 1740

tccatcaaaa aatggctttc gctacctgga gagacgcgcc cgctgatcct ttgcgaatac 1800

gcccacgcga tgggtaacag tcttggcggt ttcgctaaat actggcaggc gtttcgtcag 1860

tatccccgtt tacagggcgg cttcgtctgg gactgggtgg atcagtcgct gattaaatat 1920

gatgaaaacg gcaacccgtg gtcggcttac ggcggtgatt ttggcgatac gccgaacgat 1980
cgccagttct gtatgaacgg tctggtcttt gccgaccgca cgccgcatcc agcgctgacg 2040
gaagcaaaac accagcagca gtttttccag ttccgtttat ccgggcaaac catcgaagtg 2100
accagcgaat acctgttccg tcatagcgat aacgagctcc tgcactggat ggtggcgctg 2160
gatggtaagc cgctggcaag cggtgaagtg cctctggatg tcgctccaca aggtaaacag 2220
ttgattgaac tgcctgaact accgcagccg gagagcgccg ggcaactctg gctcacagta 2280
cgcgtagtgc aaccgaacgc gaccgcatgg tcagaagccg ggcacatcag cgcctggcag 2340
cagtggcgtc tggcggaaaa cctcagtgtg acgctccccg ccgcgtccca cgccatcccg 2400
catctgacca ccagcgaaat ggatttttgc atcgagctgg gtaataagcg ttggcaattt 2460
aaccgccagt caggctttct ttcacagatg tggattggcg ataaaaaaca actgctgacg 2520
ccgctgcgcg atcagttcac ccgtgcaccg ctggataacg acattggcgt aagtgaagcg 2580
acccgcattg accctaacgc ctgggtcgaa cgctggaagg cggcgggcca ttaccaggcc 2640
gaagcagcgt tgttgcagtg cacggcagat acacttgctg atgcggtgct gattacgacc 2700
gctcacgcgt ggcagcatca ggggaaaacc ttatttatca gccggaaaac ctaccggatt 2760
gatggtagtg gtcaaatggc gattaccgtt gatgttgaag tggcgagcga tacaccgcat 2820
ccggcgcgga ttggcctgaa ctgccagctg gcgcaggtag cagagcgggt aaactggctc 2880
ggattagggc cgcaagaaaa ctatcccgac cgccttactg ccgcctgttt tgaccgctgg 2940
gatctgccat tgtcagacat gtataccccg tacgtcttcc cgagcgaaaa cggtctgcgc 3000
tgcgggacgc gcgaattgaa ttatggccca caccagtggc gcggcgactt ccagttcaac 3060
atcagccgct acagtcaaca gcaactgatg gaaaccagcc atcgccatct gctgcacgcg 3120
gaagaaggca catggctgaa tatcgacggt ttccatatgg ggattggtgg cgacgactcc 3180
tggagcccgt cagtatcggc ggaattccag ctgagcgccg gtcgctacca ttaccagttg 3240
gtctggtgtc aaaaataata ataaccgggc aggccatgtc tgcccgtatt tcgcgtaagg 3300
aaatccatta tgtactattt aaaaaacaca aacttttgga tgttcggttt attctttttc 3360
ttttactttt ttatcatggg agcctacttc ccgtttttcc cgatttggct acatgacatc 3420
aaccatatca gcaaaagtga tacgggtatt atttttgccg ctatttctct gttctcgcta 3480
ttattccaac cgctgtttgg tctgctttct gacaaactcg gaacttgttt attgcagctt 3540
ataatggtta caaataaagc aatagcatca caaatttcac aaataaagca tttttttcac 3600
tgcattctag ttgtggtttg tccaaactca tcaatgtatc ttatcatgtc tg 3652

## Description

The present invention relates to a gene trapping construct which causes conditional mutations in genes, and the use of this gene trapping construct to mutationally inactivate all cellular genes. In addition the invention relates to a cell, preferably a mammalian cell which contains the above mentioned construct. Moreover, the invention relates to the use of said cell for identification and/or isolation of genes and for the creation of transgenic organisms to study gene function at various developmental stages, including the adult. In conclusion, the present invention provides a process which enables a temporally and/or spatially restricted inactivation of all genes that constitute a living organism.

For some years targeted mutagenesis in totipotent mouse embryonic stem (ES) cells has been used to inactivate genes for which cloned sequences were available (Capecchi, The new mouse genetics: altering the genome by gene targeting, Trends in Genetics, 5, 70 - 76 (1989)). Since ES cells can pass mutations induced in vitro to transgenic offspring in vivo it is possible to analyze the consequences of gene disruption in the context of the entire organism. Thus, numerous mouse strains with functionally inactivated genes ("knock out mice") have been created by this technology and utilized to study the biological function of a variety of genes. However, targeted mutagenesis requires detailed knowledge of gene structure and organization as well as its physical isolation in a cloning vector. Moreover, the targeting vector containing the gene of interest needs to be transduced into ES cells to isolate mutant ES clones in which a normal allele is replaced by a mutant allele by homologous recombination (Capecchi 1989). Overall, the generation of mutant mouse strains by this procedure is time consuming, labor intensive, expensive and inefficient because it can handle only one gene at the time (Koller and Smithies, Altering genes in animals by gene targeting. Ann. Rev. Immunol. 10, 705 - 730 (1992)). More importantly, mouse mutants created by this procedure (also known as "conventional, complete or classical mutants"), contain the inactivated gene in all cells and tissues throughout life.

A refined method of targeted mutagenesis, referred to as conditional mutagenesis, employs a site specific recombination system (e.g. Cre/loxP or Flp/frt - Sauer and Henderson, Site-specific recombination in mammalian cells by the Cre recombinase of bacteriophage P1, Proc. Natl. Acad. Sci. USA 85, 5166-5170 (1988); Senecoff et al., DNA recognition by the FLP recombinase of the yeast 2m plasmid: a mutational analysis of the FLP binding site , J. Mol. Biol.,201, 405 - 421 (1988)) which enables a temporally and/or spatially restricted inactivation of target genes (Rajewsky et al., Conditional gene targeting, J. Clin. Invest., 98, 600 - 603 (1996)). The creation of conditional mouse mutants requires the generation of two mouse strains, i.e. the recombinase recognition strain and the recombinase expressing strain. The recombinase recognition strain is generated by homologous recombination in ES cells as described above except that the targeted exon(s) is (are) flanked by two recombinase recognition sequences (hereinafter "RRS"), e.g. loxP or frt. Since the RRS reside in introns they do not interfere with gene expression. The recombinase expressing strain contains a recombinase transgene (e.g. Cre, Flp) whose expression is either restricted to certain cells and tissues or is inducible by external agents. Crossing of the recombinase recognition strain with the recombinase expressing strain deletes the RRS-flanked exons from the doubly transgenic offspring in a prespecified temporally and/or spatially restricted manner. Thus, the method allows the temporal analysis of gene function in particular cells and tissues of otherwise widely expressed genes. Moreover, it enables the analysis of gene function in the adult organism by circumventing embryonic lethality which is frequently the consequence of gene inactivation. For pharmaceutical research, aiming to validate the utility of genes and their products as targets for drug development, inducible mutations provide an excellent genetic tool. However, as is the case for conventional mutants, the generation of conditional mutants is a time consuming and labor intensive procedure which must be performed individually for every gene.

To address some of the problems encountered with targeted mutagenesis, several types of vectors referred to as "gene traps" have been developed that insert a promoterless reporter gene into mostly random chromosomal sites, including transcriptionally active regions (reviewed in Hill and Wurst, Mutagenic strategies to dissect mammalian development Curr. Topics. Dev. Biol. 28, 181-206 (1993); Gossler and Zachgo, Gene and enhancer trap screens in ES cell chimaeras, in: Gene targeting - a practical approach (A.L. Joyner, Ed.), Oxford University Press, Oxford (1993)). Integrations into the exons (exon traps) or introns (intron traps) of a cellular gene dirsupt the gene and generally lead to its functional inactivation. Moreover, the gene trap provides a molecular tag to clone any gene linked to a specific function. Thus, unlike homologous recombination, gene trapping is not restricted to genes for which cloned sequences are available and can be used to disrupt any gene that resides in the genome. In most cases, gene traps are transduced as retroviruses, although electroporated DNA is also used. Retroviruses have the advantage that they integrate by a precise mechanism into the genome and cause no damage to the neighbouring DNA. Moreover, unlike electroporated DNA, retroviruses almost never integrate in tandem. Since gene trap integrations are distributed randomly throughout the genome, it is possible to create mutations in a large number of genes within a a limited number of experiments (Zambrowicz et al., Disruption and sequence identification of 2,000 genes in mouse embryonic stem cells, Nature 392, 608-611 (1998)). In principle, this enables the mutational analysis of all genes within a genome by simply generating an integration library in which every single gene is disrupted by a gene trap. In most cases murine embryonic stem (ES) cells are used as targets for gene trap integrations since mutant clones can be easily obtained in vitro and passaged to transgenic offspring in vivo (Gossler et al., Mouse embryonic stem cells and reporter constructs to detect developmentally regulated genes, Science, 244, 463 - 465 (1989); Friedrich and Soriano, Promoter traps in embryonic stem cells: a genetic screen to identify and mutate developmentally genes in mice, Genes & Development, 5, 1513 - 1523 (1991); Skarnes et al., A gene trap approach in mouse embryonic stem cells: the lacZ reporter is activated by splicing, reflects endogenous gene expression, and is mutagenic in mice, Genes Dev. 6, 903-918 (1992); von Melchner et al., Selective disruption of genes expressed in totipotent embryonal stem cells, Genes & Dev 6, 919-927 (1992)). Mouse mutants obtained with this technology often develop a "loss of function phenotype" which sometimes will disclose the biological significance of a particular gene (Evans et al., Gene trapping and functional genomics, Trends in Genetics, 13, 370 - 374 (1997)). However, all currently employed gene traps induce an irreversible inactivation of their target. Consequently, a mouse mutant generated from an ES cell clone, is not different from a knock-out mouse obtained by conventional gene targetting.

The present invention combines conditional gene targeting with gene trap mutagenesis. A conditional gene trap vector typically integrates into an intron without interfering with gene function. However, activation of the gene trap by a site specific recombinase induces gene disruption and loss of its function. Unlike conditional gene targeting, the strategy is not limited to known genes and can be used to disrupt all genes in a temporally and/or spatially restricted manner.

The present invention provides
(1) a gene trapping construct capable of causing conditional mutations in genes which comprises a functional DNA segment inserted in antisense direction relative to the gene to be trapped, said functional DNA segment being flanked by two recombinase recognition sequences (RRSs) which are specific to a site specific recombinase capable of inverting a double stranded DNA segment;
(2) a preferred embodiment of the gene trapping construct defined in (1) above, which comprises two functional DNA segments,
   (a) a first DNA segment (disruption cassette) being inserted in antisense orientation relative to the transcriptional orientation of the gene to be trapped and being flanked by two RRSs which are specific to a site specific recombinase capable of inverting a double stranded DNA segment, and
   (b) a second segment (selection cassette) being positioned in sense direction relative to the transcriptional orientation of the gene to be trapped and being flanked by two RRSs in the same orientation;
(3) a cell comprising the trapping construct as defined in (1) or (2) above;
(4) use of the cell of (3) above for the identification and/or isolation of genes;
(5) a process for the generation of conditional mutations in all genes of an organism comprising
   (i) installation of a gene trapping construct as defined in (2) above in a suitable cell,
   (ii) selection of cells in which the construct is incorporated in a gene,
   (iii) identification and/or isolation of the gene in which the construct is incorporated,
   (iv) deletion of the selection cassette from the trapped gene,
   (v) induction of a mutation in the trapped gene by inversion of the gene disruption cassette;
(6) a transgenic organism obtainable by the method of (5) above; and
(7) use of the transgenic organism according to (6) above to study gene function at various developmental stages.

### Short description of Figures

Fig. 1: Conditional gene trap vector to modify genes expressed in the target cells.

fig. 2: Conditional retroviral gene trap vector to modify expressed genes.

Fig. 3: Conditional gene trap vector to modifiy genes independently of their expression level in the target cells.

Fig. 4: Conditional retroviral gene trap vector to modify genes independently of their expression.

The gene trapping construct (1) is hereinafter described in more detail:

The activation mechanism of a conditional gene trap is based on the ability of a site specific recombinase (e.g. Cre or Flp) to invert a double stranded DNA segment flanked by two RRSs (e.g. loxP or frt) inserted in opposite orientations (Abremski et al., Studies on the properties of P1 site specific recombination: evidence for topologically unlinked products following recombination, Cell, 32, 1301 - 1311 (1983); Hamilton et al., Site specific recombination of the bacteriophage P1 lox-Cre system, Cre-mediated synapsis of two lox sites, J. Mol. Biol., 178, 481- 486 (1984); Albert et al., Site-specific integration of DNA into wild-type and mutant lox sites placed in the plant genome, The Plant Journal, 7, 649 - 659 (1995); Vetter et al., Site-specific recombination of yeast 2-µm DNA in vitro, Proc. Natl. Acad. Sci. USA, 80, 7284 - 7288 (1983)). If this is used in the context of a gene trap vector, sequence elements that interfere with gene expression (e.g. splice sites, polyadenylation signals) can be first inserted into an intron in an antisense direction relative to the gene. By residing on the non-transcribed DNA strand, these elements will not interfere with gene transcription. Whenever or wherever a gene disruption is desired, inversion is induced by expressing the appropriate site specific recombinase.

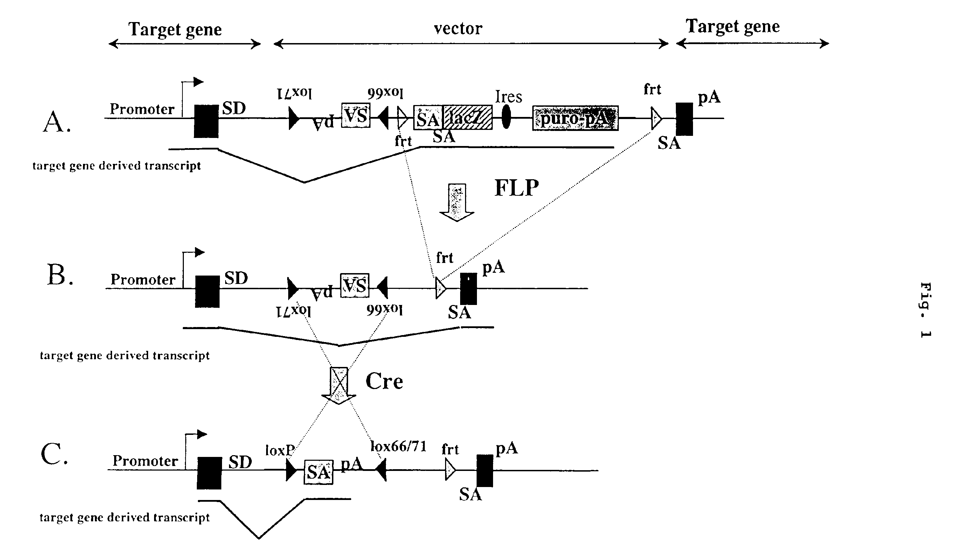

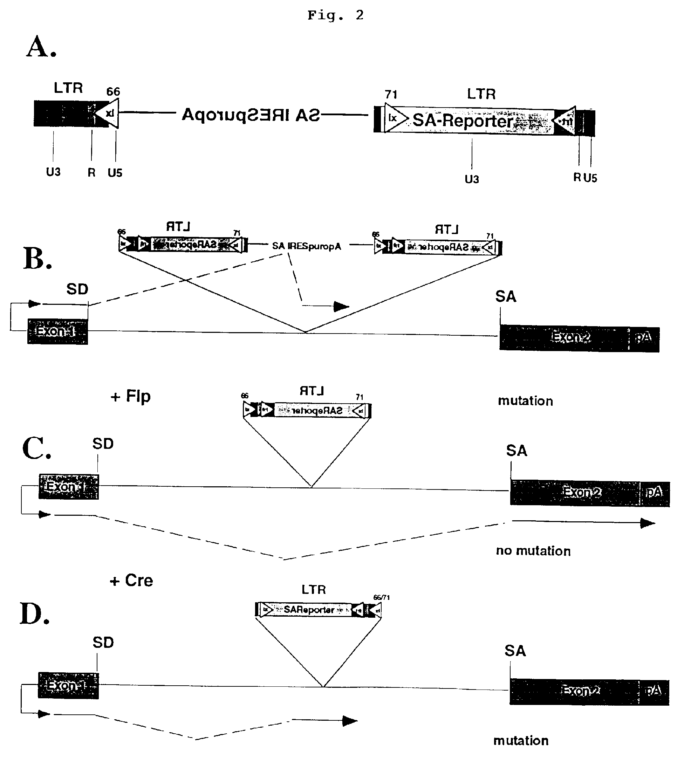

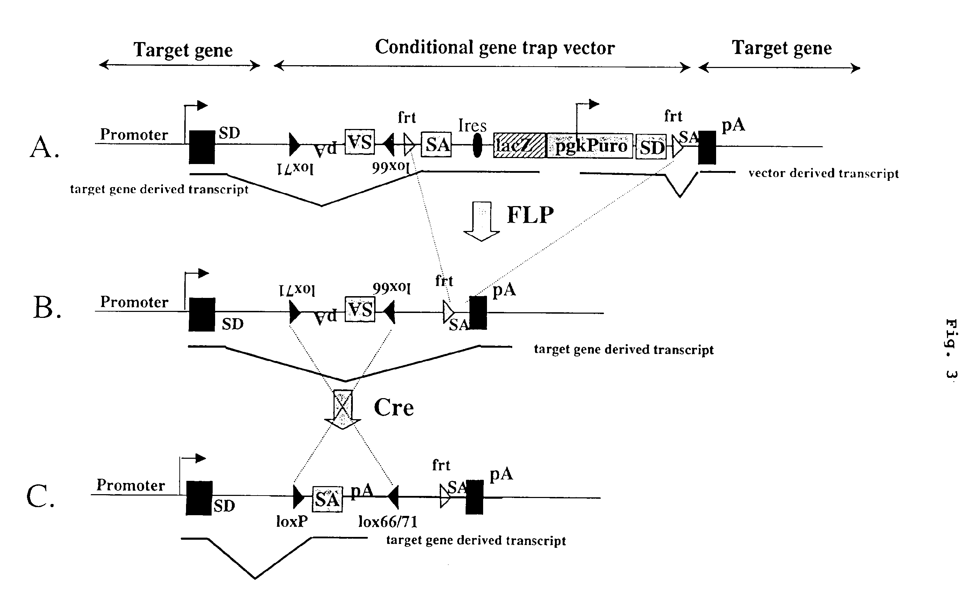

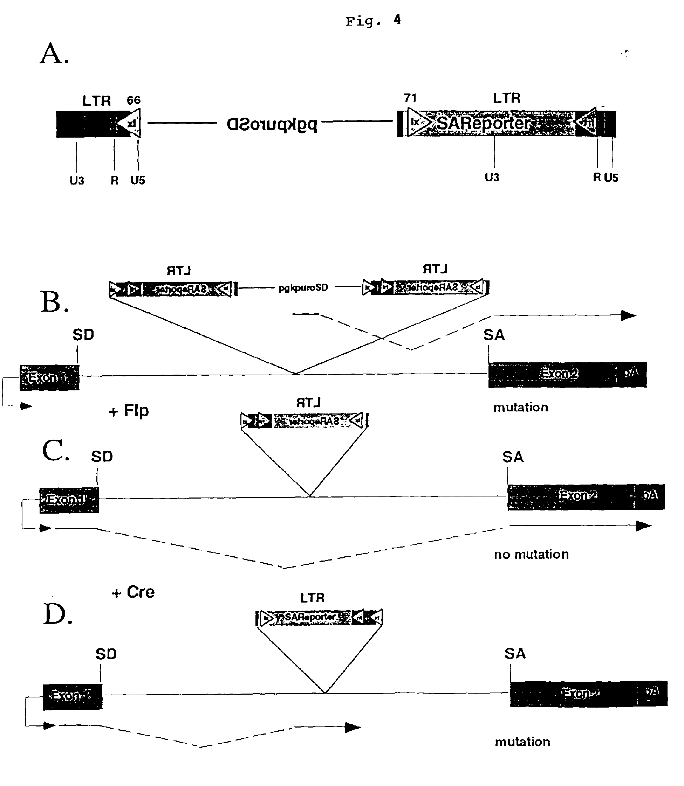

The preferred types of operation with respect to the invention are plasmid (Figures 1 and 3) or retroviral vectors (Figures 2 and 4) which can select for integrations into introns. Selection is based on the expression of a reporter gene that requires either an active cellular promoter (expressed genes) (Figures 1 and 2) or the acquisition of a endogenous transcriptional termination (polyadenylation) signal (Figures 3 and 4).

The principal elements of a conditional gene trap vector that selects for integrations into expressed genes are (i) a conditional gene disruption cassette, containing a 3' splice site (splice acceptor; SA; see SEQ ID NO:1) and a polyadenylation sequence (polyA) flanked by two RRSs (RRS1) of a site specific recombinase (rec1 ) in opposite orientation, and (ii) a selection cassette containing a reporter or selectable marker gene flanked by an upstream SA- and a downstream polyA- site. The selection cassette is flanked by two RRSs (RRS2) in same orientation which are recognized by second recombinase (rec2) and is in opposite orientation to the gene disruption cassette (Figures 1 and 3). Selection for gene expression yields recombinants in which the reporter gene is fused to the regulatory elements of an endogenous gene. Transcripts generated by these fusions encode for a truncated cellular protein which has lost its normal function. Since selection for a gene trap event relies on the expression of the selection cassette which is by itself mutagenic, it needs to be removed to recreate gene function. This is achieved by expressing rec2 in recombinants selected for gene trap integrations. In a favoured operational process the conditional gene trap is transduced into ES cells. After selecting for integrations into the introns of expressed genes, rec2 is transiently expressed in individual clones to delete the selection cassette to restore gene function. The resulting clones containing only the gene disruption cassette are used to create transgenic mouse strains. Such mouse strains are crossed to rec1 expressing mouse strains to obtain doubly transgenic offspring where the gene disruption cassette is reversed to its mutagenic (sense) orientation.

As a further preferred embodiment the invention provides a conditional gene trap vector that selects for integrations into all genes. This is achieved by replacing the elements of the original selection cassette by a reporter gene that is fused to an upstream constitutive promoter and to a downstream 5'splice site (splice donor) (Zambrowicz, et al., 1998) (Figures 3 and 4). Expression of this gene trap is dependent on the acquisition of an endogenous polyadenylation sequence which occurs by splicing of the selection cassette to the donwstream exons of the target gene. Since the process is driven by a constitutive promoter, selection for gene trap integrations is independent of target gene expression. As with the other conditional gene trap, a favoured operational process is its transduction into ES cells and the generation of mutant mouse strains.

The present invention further provides a site specific recombination system which enables an unidirectional inversion of sequences flanked by RRS1 sites. Conventional site specific recombinases normally recombine reversibly between identical recognition sites (like two loxP or FRT sites). In a conditional gene trap setting this would mean that equal amounts of sense and antisense products would be generated whereby one half of the target alleles carrying the gene trap would be inactivated whereas the other half would still have a functional configuration. Since most genes of the mammalian genome are recessive, inactivation of one allele is normally insufficient to produce an observable phenotype. Therefore, it is important to shift the equilibrium of the recombinase reaction towards the gene trap inversion that causes gene inactivation. This is achieved by using mutant RRSs which recombine only once. For example, in the Cre/loxP recombination system, the recombination between the two single mutant loxP recognition sites (e.g. lox66 and lox71; Albert et al, 1995; see SEQ ID NOs:2 and 3) generates a double mutant- and a wildtype- loxP site (see SEQ ID NOs:4 and 5), each on one side of the inverted DNA. Since this combination of loxP sites is less efficiently recognised by the Crerecombinase, the inversion is mostly unidirectional. Alternatively, recombinases which normally recognize nonidentical RRSs, such as the phage derived integrases of the integrase (Nunes-Düby et al., Similarities and differences among 105 members of the Int family of site-specific recombinases, Nucleic Acid Res. 26, 391 - 406 (1998)) or resolvase (Thorpe et al. In vitro site-specific integration of bacteriophage DNA catalyzed by a recombinase of the resolvase/invertase family, Proc. Natl. Acad. Sci. USA 95, 5505 - 5510 (1998)) family of recombinases, may be used for gene trap inversion.

In conclusion, the present invention also provides a process for the generation of conditional mutations in all genes of an organism and contains the following steps:
(i) Installation of a gene trapping construct in a suitable cell
(ii) Selection of cells in which the construct is incorporated in a gene
(iii) Identification and/or isolation of the gene in which the construct is incorporated
(iv) Deletion of the selection cassette from the trapped gene
(v) Induction of a mutation in the trapped gene by inversion of the gene disruption cassette.

The above process possesses the following advantages over current technology:
(i) mutations are inducible in prespecified cells and tissues and during prespecified time intervals;
(ii) mutations can be induced in all genes, including those for which cloned sequences are not available;
(i) the functional analysis of the mutant genes in appropriate organisms is relatively fast and cheap.

The appended figures further explain the present invention:

Figure 1 shows a favoured operational form of the process relating to a conditional gene trap transduced by electroporation to capture expressed genes.
A: Target gene after insertion of a conditional gene trap vector into an intron of an expressed gene. In transcripts initiating at the target gene's promoter the LacZ- and the puromycin resistance genes (selection cassette) are fused to the upstream exons by a splice acceptor site. The fusion disrupts and inactivates the target gene.
B: Modified allele after FLP mediated deletion of the selection cassette. Excision of sequences flanked by the frt recognition sites which include the splice acceptor signal restores wild type transcription and gene function.
C: Target gene after Cre mediated inversion of the loxP flanked gene trap elements (gene disruption cassette). Gene trap inversion positions the splice acceptor- and the poly A- sites into the sense orientation which is mutagenic.
SA: splice acceptor signal sequence; SD: splice donor signal sequence; pA: polyadenylation sequence; lacZ: beta-Galactosidase; puro: puromycin resistance gene; filled rectangles: exons; filled triangles: loxP recognition sites, loxP: wildtype loxP site, lox66: single mutant lox site, lox71: single mutant lox site; lox 66/71: double mutant lox site; shaded triangles: FRT recognition sites (frt); thick continuous lines: mRNA transcripts; thin continuous lines: intron; thin stippled lines: recombinase mediated deletion or inversion between lox or FRT sites. Cre: Cre recombinase; FLP: FLP recombinase. Ires: Internal ribosomal entry site.

Figure 2 shows a favoured operational form of the process relating to a conditional gene trap transduced by retroviruses to capture expressed genes.
A: Retroviral plasmid vector
B. Target gene after insertion of a conditional gene trap provirus into an intron of an expressed gene. Virus replication and LTR mediated duplication places the gene disruption cassette in U3 between mutant loxP sites in both 5' and 3' proviral LTRs. Moreover, it places the selection cassette inserted into the body of the virus between two frt sites. Transcripts initiating in the cellular promoter are spliced to the selection cassette by an upstream splice acceptor site. This fusion disrupts and inactivates the target gene.
C: Modified allele after FLP mediated excision of all proviral sequences placed between the frt sites. This restores gene function and leaves only one copy of gene disruption cassette behind.
D: Target gene after Cre mediated inversion of the loxP flanked gene trap elements. LTR inversion positions the gene disruption cassette into a mutagenic sense orientation.
LTR = long terminal repeat, U3 = 3' unique region of the retroviral LTR, R = repetitive region of the retroviral LTR, U5 = 5' unique region of the retroviral LTR, SA = splice acceptor signal sequence; SD = splice donor sequence; pA = polyadenylation sequence; Puro = puromycin resistance gene; lx = loxP recognition sites; frt = Flp recognition sites; thin continuous lines = intron; thin stippled lines = sequences excised by mRNA processing; arrows indicate transcriptional orientation.

Figure 3 shows a favoured operational form of the process relating to a conditional gene trap transduced by electroporation to capture all genes.
A: Target gene after insertion of a conditional gene trap vector into an intron of a gene. Transcripts initiating in the independent pgk promoter express the puromycin resistance gene after splicing into downstream exons to acquire a polyadenylation site. Splicing is mediated by a splice donor sequence inserted downstream of pgkpuro. The fusion of the selection cassette to downstream exons inactivates the target gene.
B: Modified allele after FLP mediated deletion of the selection cassette which restores gene function.
C: Target gene after Cre mediated inversion of the gene disruption cassette which causes the mutation.
SA: splice acceptor signal sequence; SD: splice donor signal sequence; pA: polyadenylation sequence; lacZ: beta-Galactosidase; puro: puromycin resistance gene; filled rectangles: exons; filled triangles: loxP recognition sites, loxP: wildtype loxP site, lox66: single mutant lox site, lox71: single mutant lox site; lox 66/71: double mutant lox site; shaded triangles: FRT recognition sites (frt); thick continuous lines: mRNA transcripts; thin continuous lines: intron; thin stippled lines: recombinase mediated deletion or inversion between lox or FRT sites. Cre: Cre recombinase; FLP: FLP recombinase. Ires: Internal ribosomal entry site; pgk: phosphoglyceratekinase promoter. .

Figure 4 shows a favoured operational form of the process relating to a conditional gene trap transduced by retroviruses to capture all genes.
A: Retroviral plasmid vector
B. Target gene after insertion of a conditional gene trap provirus into an intron of a gene.
   Virus replication and LTR mediated duplication places the gene disruption cassette in U3 between mutant loxP sites in both 5'and 3' proviral LTRs. Moreover, it places the selection cassette inserted into the body of the virus between two frt sites. Transcripts initiating in the independent pgk promoter express the puromycin resistance gene after splicing into downstream exons to acquire a polyadenylation site. Splicing is mediated by a splice donor sequence inserted downstream of pgkpuro. The fusion of the selection cassette to the downstream exons inactivates the target gene.
C: Modified allele after FLP mediated excision of all proviral sequences placed between the frt sites. This restores gene function and leaves only one copy of gene disruption cassette behind.
D: Target gene after Cre mediated inversion of the loxP flanked gene trap elements. LTR inversion positions the gene disruption cassette into a mutagenic sense orientation.
LTR = long terminal repeat, U3 = 3' unique region of the retroviral LTR, R = repetitive region of the retroviral LTR, U5 = 5' unique region of the retroviral LTR, SA = splice acceptor signal sequence; SD = splice donor sequence; pA = polyadenylation sequence; Puro = puromycin resistance gene; lx = loxP recognition sites; frt = Flp recognition sites; thin continuous lines = intron; thin stippled lines = sequences excised by mRNA processing; pgk = phosphoglyceratekinase promoter; arrows indicate the direction of transcript elongation.

## Claims

1. A gene trapping construct capable of causing conditional mutations in genes which comprises a functional DNA segment inserted in antisense direction relative to the gene to be trapped, said functional DNA segment being flanked by two recombinase recognition sequences (RRSs) which are specific to a site specific recombinase capable of inverting a double stranded DNA segment.

2. The gene construct according to claim 1, wherein the RRSs are loxP or frt sequences in opposite orientation, and the site specific recombinase is Cre or Flp, respectively, preferably the two RRSs are single mutant lox P or frt sites in opposite orientation, most preferably the two RRSs are lox 66 and lox 71 in opposite orientation.

3. The gene construct according to claim 1, wherein the functional DNA segment further comprises one or more of the following splice acceptor, splice donor, polyadenylation sequence, a gene coding for a reporter protein, a resistance gene and a gene coding for a further site specific recombinase.

4. The gene construct according to claim 1 or 2, which further comprises a selection DNA segment suitable for selecting for genes having an incorporated gene trapping construct, said selection DNA segment comprising a reporter or resistance gene and flanking recombinase recognition sites in same orientation.

5. The gene construct according to claim 4 being a conditional gene trapping construct selecting for integrations into expressed genes, wherein the functional DNA segment comprises a splice acceptor and a polyadenylation sequence flanked by two RRSs of a first site specific recombinase, and the selection DNA segment comprises a selection cassette which comprises a reporter or selectable marker gene flanked by an upstream splice acceptor sequence and a downstream polyadenylation sequence, said selection cassette being flanked by two RRSs of a second site specific recombinase in same orientation.

6. The gene construct according to claim 4 being a conditional gene trapping construct selecting for integrations into all genes, wherein the functional DNA segment comprises a splice acceptor and a polyadenylation sequence flanked by two RRSs of a first site specific recombinase, and the selection DNA segment comprises a selection cassette which comprises a reporter or selectable marker gene fused to an upstream constitutive promoter and a downstream splice donor site, said selection cassette being flanked by two RRSs of a second site specific recombinase in same orientation.

7. A cell comprising the trapping construct as defined in claims 1 to 6.

8. Use of the cell of claim 7 for the identification and/or isolation of genes.

9. A process for the generation of conditional mutations in all genes of an organism comprising
(i) installation of a gene trapping construct as defined in claims 1 to 6 in a suitable cell,
(ii) selection of cells in which the construct is incorporated in a gene,
(iii) identification and/or isolation of the gene in which the construct is incorporated,
(iv) deletion of the selection cassette from the trapped gene,
(v) induction of a mutation in the trapped gene by inversion of the gene disruption cassette.

10. The process of claim 9 which is sutable for temporarily and/or spatially restricted inactivation of all genes that constitute a living organism.

11. The process of claim 9 or 10 being a process for preparing transgenic mammals, wherein in step (i) the gene trapping construct is installed in an ES cell.

12. A transgenic organism obtainable by the method of claims 9 to 11.

13. Use of the transgenic organism according to claim 12 to study gene function at various developmental stages.
